# FASCICULE DE BREVET EUROPEEN

(11) **EP 0 601 102 B1**
(45) Date de publication et mention de la délivrance du brevet: **20.03.1996**
(21) Numéro de dépôt: 92919408.2
(22) Date de dépôt: 28.08.1992
(51) Int. Cl.: A61K 7/00, A61K 7/48, A61K 7/02, A61K 7/035

(54) **PARTICULES REVETUES DE PIGMENT, UTILES DANS DES PREPARATIONS COSMETIQUES, ET PROCEDE POUR LEUR PREPARATION**
MIT PIGMENT BESCHICHTETE PARTIKEL ZUR VERWENDUNG IN KOSMETISCHEN ZUSAMMENSETZUNGEN, SOWIE VERFAHREN ZU DEREN HERSTELLUNG
PIGMENT-COATED PARTICLES FOR USE IN COSMETIC COMPOSITIONS AND PROCESS FOR THE PREPARATION OF SAID PARTICULES

(30) Priorité: 29.08.1991 US 751701
(43) Date de publication de la demande: 15.06.1994
(73) Titulaire: L'OREAL, F-75008 Paris (FR)
(72) Inventeur: FARER, Alan, M., Morganville, NJ 07751 (US); BURDZY, Elisa, L., Clifton, NJ 07013 (US); HANNA, Fifi, Kearny, NJ 07032 (US); PENICNAK, A. John, Mountain Lakes, NJ 07046 (US); MELLUL, Myriam, F-94240 L'Hay-les-Roses (FR)
(74) Mandataire: Stalla-Bourdillon, Bernard
(86) Numéro de dépôt international: FR9200831
(87) Numéro de publication internationale: WO9304660

(56) Documents cités:
- EP-A- 0 254 612
- EP-A- 0 379 409
- EP-A- 0 447 286
- EP-A- 0 486 394
- FR-A- 2 594 130
- US-A- 5 106 838

## Description

L'invention concerne des particules substrat polymères revêtues d'un pigment, pour l'emploi dans des compositions cosmétiques, comprenant un agent de couplage silane ou titanate fixé par covalence à la surface extérieure des particules substrat et un revêtement de pigment fixé par covalence à l'agent de couplage. L'invention concerne également des compositions cosmétiques contenant les particules revêtues de pigment, ainsi gu'un procédé pour leur préparation.

Des préparations cosmétiques ont été utilisées depuis très longtemps pour améliorer l'aspect de la peau et des cheveux. Bien que la plupart des produits cosmétiques aient des compositions relativement simples, ils contiennent souvent des ingrédients qui sont incompatibles et difficiles à mettre en oeuvre.

Par exemple, des composants tels que des pigments ne se dispersent pas facilement dans les produits avec des phases d'huile et d'eau. Ces problèmes sont particulièrement importants dans le cas des compositions pigmentées, telles qu'un fard à paupières ou un fard à joues en plaquette qui contiennent des matières en particules et nécessitent une préparation minutieuse pour l'obtention de caractéristiques colorées satisfaisantes. Cela exige un mélange, un broyage et un tamisage coûteux en main-d'oeuvre et en énergie.

Pour améliorer la dispersion et les caractéristiques apparentées, on a revêtu de titanates les matières cosmétiques (US 4 877 604). Ce brevet décrit, entre autres, le revêtement de pigments avec des titanates organiques.

On a découvert que, de façon surprenante, des particules polymères revêtues de pigment peuvent être obtenues par emploi de microsphères creuses d'un copolymère de polyvinylidène.

L'utilisation de microsphères creuses non revêtues d'un copolymère de polyvinylidène du type chlorure de vinylidène/acrylonitrile a déjà été décrite dans EP 254.612 en vue d'améliorer les propriétés cosmétiques de poudres non compactées.

La présente invention a donc pour objet, pour l'emploi dans des compositions cosmétiques, des particules revêtues de pigment comprenant chacune une particule substrat polymère, un agent de couplage silane ou titanate fixé par covalence à la surface extérieure de la particule substrat polymère et un revêtement de pigment fixé par covalence à l'agent de couplage, la particule substrat polymère étant caractérisée en ce qu'elle est une microsphère creuse d'un copolymère de polyvinylidène.

L'emploi de microsphères creuses de copolymère de polvvinylidène selon l'invention fournit des particules revêtues de pigment qui sont plus faciles à mettre en oeuvre et qui nécessitent moins de pigment, ce qui conduit à des économies de matières premières.

Un avantage particulier de la présente invention est que les étapes de mélange et de broyage, normalement nécessaires pour assurer la dispersion appropriée des pigments dans les véhicules cosmétiques, deviennent inutiles.

De plus, les particules revêtues de pigment de l'invention, qui sont notablement plus grosses que les particules de pigment libre, ont moins tendance à obturer le canal pilosébacé.

Un autre avantage est que, par rapport au pigment libre, les particules revêtues de pigment de l'invention ont une masse volumique apparente réduite, si bien que, lorsqu'on les ajoute à des préparations cosmétiques fluides, telles que des émulsions, elles ont bien moins tendance à se séparer, ce qui évite une altération de la couleur ou une striation du produit.

De plus, les particules revêtues de pigment de l'invention présentent une amélioration inattendue de la couleur par rapport au même pigment sous la forme libre.

En fait, lorsqu'on les incorpore à des compositions cosmétiques, les particules revêtues de pigment de l'invention établissent une intensité de couleur et une homogénéité exceptionnelles dans des préparations telles que les produits en poudre, les mascaras, les émulsions (y compris les produits de maquillage et les produits hydratants), les rouges à lèvres, les eye-liners, les contours de lèvres, les fards à joues en crème, les vernis à ongles et les produits de maquillage en crème.

Les particules revêtues de pigment de l'invention ont généralement une granulométrie moyenne dans la gamme de 5 à 250 µm, de préférence de 5 à 150 µm, tout préférablement de 5 à 80 µm.

Les microsphères creuses non revêtues ont en général une granulométrie moyenne, sous la forme libre, de 5 à 40 µm, de préférence de 5 à 25 µm.

Le pigment utilisé selon l'invention peut avoir une granulométrie moyenne, sous la forme libre, de 0,01 à 25 µm, de préférence de 0,02 à 5 µm.

On préfère que les microsphères creuses aient un diamètre moyen au moins double, de préférence au moins décuple du diamètre moyen des particules de pigment libre.

Selon l'invention, le pigment peut être présent à raison de 40 à 97 %, de préférence de 75 à 90 % en poids, relativement au poids des particules revêtues de pigment.

Les pigments selon l'invention sont ceux habituellement utilisés dans les compositions cosmétiques et sont choisis en particulier parmi les pigments minéraux et organiques.

Les pigments minéraux comprennent par exemple :
- le dioxyde de titane (rutile ou anatase), éventuellement traité en surface et codifié dans le Color Index sous la référence CI 77891 ;
- les oxydes ferreux noir, jaune, rouge et brun, codifiés sous les références CI 77499, 77492 et 77491 ;
- l'oxyde de zinc ou le dioxyde de zirconium ;
- le violet de manganèse (CI 77742) ;
- le bleu d'outremer (CI 77007) ;
- l'oxyde de chrome (CI 77288) ;
- l'oxyde de chrome hydraté (CI 77289) ; et
- le Bleu ferrique (CI 77510).

Les pigments organiques comprennent les suivants :
- D & C red n° 19 (CI 45170) ;
- D & C red n° 9 (CI 15585) ;
- D & C red n° 21 (CI 45380) ;
- D & C orange n° 4 (CI 15510) ;
- D & C orange n° 5 (CI 45370) ;
- D & C red n° 27 (CI 45410) ;
- D & C red n° 13 (CI 15630) ;
- D & C red n° 7 (CI 15850:1) ;
- D & C red n° 6 (CI 15850:2) ;
- D & C yellow n° 5 (CI 19140) ;
- D & C red n° 36 (CI 12085) ;
- D & C orange n° 10 (CI 45425) ;
- D & C yellow n° 6 (CI 15985) ;
- D & C red n° 30 (CI 73360) ;
- D & C red n° 3 (CI 45430) ;
- le noir de four (CI 77266) ; et
- les laques carmin de cochenille (CI 75470) ; et
- la mélanine synthétique ou naturelle.

L'agent de couplage silane et/ou titanate, convenant à l'emploi dans l'invention, est, dans le premier cas, de préférence un trialcoxysilane et, dans le second cas, de préférence un monoalkyltitanate (souvent appelé également monoalcoxytitanate) ou un produit de coordination phospho-titanate.

Les trialcoxysilanes (notamment triméthoxy et triéthoxysilanes) utilisables sont en particulier les alkyltrialcoxysilanes et les perfluoroalkyltrialcoxysilanes dont les groupements alkyle ont notramment 1 à 20 atomes de carbone, par exemple l'isobutyltriméthoxysilane.

Des monoalkyl titanates utiles comprennent les monoalkyl triisostéaroyl titanates, les monoalkyl diisostéaroyl méthacryloyl titanates, les monoalkyl isostéaroyl diméthacryloyl titanates et les monoalkyl triméthacryloyl titanates, dont le groupe alkyle a par exemple de 1 à 20 atomes de carbone.

Ou utilise de préférence l'isopropyl isostéaroyl diméthacryloyl titanate ou l'isopropyl triisostéaroyl titanate, ce dernier étant tout particulièrement préféré.

Des produits de coordination de type phospho-titanate utiles comprennent le di(dioctyl)phosphitotitanate de tétra-isopropyle et le di( ditridécyl)phosphitotitanate de tétra(2,2-diallyloxyméthyl)butyle.

L'agent de couplage est normalement liquide à la température ordinaire et peut être appliqué tel quel aux microsphères creuses. Sinon, avant l'emploi, l'agent de couplage peut être dissous ou dilué dans l'eau ou dans un solvant organique, tel que le benzène, le toluène ou la butane-2-one.

Selon l'invention les microsphères creuses de copolymère de polyvinylidène ont de préférence une masse volumique de 0,01 à 0,1, mieux de 0,01 à 0,065 g/cm³. La partie creuse des microsphères peut être remplie d'un gaz, typiquement un hydrocarbure, de préférance l'isobutane, qui constitue de 5 à 30 % p/p des microsphères. Les microsphères creuses peuvent être préparées selon le procédé décrit dans la demande de brevet européen 056 219 et sont tout préférablement faites d'un copolymère de chlorure de vinylidène/acrylonitrile, telles que celles fournies parla Société Kemanord Plast sous le nom commercial d'Expancel 551 DE 20 (granulométrie moyenne : 25 µm) et d'Expancel 551 DE (granulométrie moyenne : 40 µm). La granulométrie moyenne est mesurée par diffraction laser avec un Malvern Mastersizer®.

La présente invention concerne également des compositions cosmétiques contenant les particules revêtues de pigment exposées ci-dessus, de préférence à raison de 0,05 à 90 et mieux de 0,5 à 50 % p/p de la composition.

Ces compositions peuvent être sous l'une quelconque des formes de cosmétiques mentionnées ci-dessus et peuvent contenir des ingrédients classiques utilisés dans les cosmétiques, tels que des pigments, des charges, des huiles, des cires, des liants, des humectants, des parfums, des conservateurs, des filtres solaires, etc.

La composition cosmétique peut également contenir une seconde matière en particules revêtue, telle que du talc revêtu de silicone, d'amino-acides, de lécithine ou de collagène.

La présente invention concerne également un procédé pour la préparation des particules revêtues de pigment exposées ci-dessus. Ce procédé est caractérisé en ce que l'on traite tout d'abord les microsphères creuses avec 0,01 à 5,0 % p/p, relativement aux microsphères creuses, d'un agent de couplage silane ou titanate, avec agitation continue, pour former un mélange homogène auquel on ajoute une suspension fluide d'au moins un pigment et, après poursuite de l'agitation, on sépare les particules revêtues de pigment.

Dans ce procédé, la suspension fluide du pigment est de préférence une suspension aqueuse et l'agent de couplage est de préférence ajouté à une suspension aqueuse des microsphères creuses non revêtues. On sépare ensuite, par exemple par filtration, les particules revêtues de pigment.

Un mode de mise en pratique de ce procédé consiste à ajouter environ 2 % (p/p relativement aux microsphères creuses) d'un agent de couplage liquide à une dispersion, ou suspension, aqueuse uniforme des microsphères creuses, à la température ordinaire, dans des conditions d'agitation rapide. Après 30 à 60 minutes d'agitation, on ajoute une suspension aqueuse du pigment au mélange en poursuivant l'agitation rapide. On agite le mélange pendant encore 20 à 60 minutes, puis on sépare par filtration les particules revêtues de pigment.

Un autre mode de réalisation du procédé selon l'invention comprend tout d'abord la pulvérisation de l'agent de couplage liquide sur un lit fluidisé ou agité des microsphères creuses, puis la dispersion des microsphères creuses traitées avec l'agent de couplage dans des conditions de mélange rapide, à la température ordinaire, avec de l'eau, pour former une suspension ou mélange aqueux. On ajoute ensuite une suspension du pigment au mélange que l'on agite rapidement à la température ordinaire pendant 20 à 60 minutes. On sépare ensuite du mélange par filtration les particules revêtues de pigment.

Les exemples suivants sont présentés pour illustrer l'invention.

### EXEMPLE 1 :

### Préparation de microsphères creuses de copolymère de polyvinylidène revêtues de pigment

On ajoute 2 g d'isopropyl triisostéaroyltitanate (ITT) à une suspension aqueuse de 20 g d'Expancel 551 DE. On mélange rapidement avec un mélangeur de type Lightin pendant environ 1 heure à la température ordinaire. Ensuite, on ajoute 78 g de pigment outremer en agitant pour revêtir uniformément le mélange. On chauffe ensuite le mélange d'Expancel/ITT/outremer pour achever la réaction et chasser une partie de l'eau. On sépare ensuite par filtration l'eau résiduelle et on soumet le produit à un séchage forcé.

### EXEMPLES 2 A 10 :

On reprend le mode opératoire de l'exemple l en utilisant, comme indiqué, de l'Expancel 551 DE 20 ou de l'Expancel 551 DE, mais en utilisant des pigments différents en les quantités indiquées dans le tableau suivant.

**TABLEAU**

| Ex. | Pigment | Expancel | Quantité en % en poids | | |
|---|---|---|---|---|---|
| | | | Pigment | ITT | Microsphères creuses |
| 2 | outremer | DE | 83 | 2 | 15 |
| 3 | D&C red n° 7 | DE | 73 | " | 25 |
| 4 | oxyde de fer rouge | DE 20 | 83 | " | 15 |
| 5 | oxyde de fer jaune | DE 20 | 78 | " | 20 |
| 6 | oxyde de fer noir | DE 20 | 78 | " | 20 |
| 7 | oxyde de fer noir | DE | 78 | " | 20 |
| 8 | oxyde de fer noir (ultrafin) | DE 20 | 83 | " | 15 |
| 9 | dioxyde de titane | DE 20 | 78 | " | 20 |
| 10 | oxyde de zinc | DE 20 | 78 | " | 20 |

### EXEMPLES DE COMPOSITIONS COSMETIQUES

### EXEMPLE A : formule de démonstration

| | |
|---|---|
| Cire de paraffine | 69 g |
| Cire de polyéthylène | 30 g |
| Particules revêtues de pigment de l'exemple 1 | 1 g |

On prépare la formule par fusion de la phase de cire, puis addition des particules revêtues de pigment en s'aidant d'une spatule. On verse ensuite le mélange dans un moule et on le laisse faire prise. La formule a une couleur rouge intense et saturée et l'examen microscopique confirme l'excellente dispersion du colorant.

### EXEMPLE B : formule de démonstration

| | |
|---|---|
| Cire de paraffine | 69 g |
| Cire de polyéthylène | 30 g |
| Particules revêtues de pigment de l'exemple 3 | 1 g |

On prépare la formule de la même façon que dans l'exemple A et le produit final a une luminosité et une saturation accrues.

### EXEMPLE C : fard à joues

| | |
|---|---|
| Huile de paraffine | 46,7 g |
| Vaseline | 10 g |
| Cire de carnauba | 5 g |
| Cire de polyéthylène | 5 g |
| Mica traité avec un titanate | 10 g |
| Poudre de nylon 12 | 10 g |
| Particules revêtues de pigment de l'exemple 4 | 3,5 g |
| Particules revêtues de pigment de l'exemple 5 | 0,8 g |
| Particules revêtues de pigment de l'exemple 9 | 8,4 g |
| Particules revêtues de pigment de l'exemple 3 | 0,4 g |
| Parahydroxybenzoate de propyle | 0,2 g |

Pour préparer ce fard à joues, on fond la phase grasse avec le conservateur, puis on ajoute la phase colorée en s'aidant d'une spatule. On ajoute ensuite le mica traité avec un titanate (pigment nacré) et la charge (poudre de nylon 12) entre 70 et 80°C. On refroidit ensuite le tout à 50°C sous agitation.

### EXEMPLE D : fond de teint

| Phase w | |
|---|---|
| Eau | qsp 100 g |
| Polymères d'acide acrylique (Carbopol 941 de Goodrich) | 0,35 g |
| Hydroxyde de sodium | 0,14 g |

| Phase x | |
|---|---|
| Particules revêtues de pigment de l'exemple 5 | 0,75 g |
| Particules revêtues de pigment de l'exemple 4 | 0,47 g |
| Particules revêtues de pigment de l'exemple 6 | 0,23 g |
| Particules revêtues de pigment de l'exemple 9 | 4,55 g |

| Phase y | |
|---|---|
| Stéarate de glycérol | 0,3 g |
| Stéarate de sodium | 0,75 g |
| Triglycérides capryliques/capriques | 3,5 g |
| Escalol 507 (filtre solaire)* | 1,0 g |
| Alcool cétylique | 0,5 g |
| Cyclopentadiméthylsiloxane | 5 g |

| | |
|---|---|
| * para-aminobenzoate de 2-éthylhexyle vendu par Van Dyk. | |

| Phase z | |
|---|---|
| Eau | 5 g |
| Glycérine | 3 g |
| Conservateurs | 0,3 g |

Pour préparer le fond de teint, on prépare d'abord le gel entre 80 et 90°C (phase w). On le neutralise ensuite avant l'addition de la phase colorée (phase x) avec agitation. On fond ensuite la phase grasse (phase y) entre 60 et 70°C et on l'ajoute au gel en utilisant un mélangeur mécanique. On ajoute ensuite les composants finals (phase z) à environ 40°C. La base de maquillage obtenue a une couleur beige doré et présente une dispersion régulière lorsqu'on l'observe au microscope. Il n'y a pas de changement de couleur ni avant ni après étalement de la base.

## Revendications

1. Particules revêtues de pigment constituées chacune d'une particule substrat polymère, d'un agent de couplage silane ou titanate fixé par covalence à la surface extérieure de la particule substrat polymère et d'un revêtement de pigment fixé par covalence à l'agent de couplage, caractérisées en ce que la particule substrat polymère est une microsphère creuse d'un copolymère de polyvinylidène.

2. Particules revêtues de pigment selon la revendication 1, caractérisées en ce que la masse volumique des microsphères creuses avant revêtement est comprise entre 0,01 et 0,1 g/cm³, de préférence entre 0,01 et 0,065 g/cm³.

3. Particules revêtues de pigment selon la revendication 1 ou 2, caractérisées en ce que la taille des microsphères creuses avant revêtement est comprise entre 5 et 40 µm, de préférence entre 5 et 25 µm.

4. Particules revêtues de pigment selon l'une quelconque des revendications précédentes, caractérisées en ce que copolymère de polyvinylidène est un copolymère de chlorure de vinylidène/acrylonitrile.

5. Particules revêtues de pigment selon l'une quelconque des revendications précédentes, caractérisées en ce que les microsphères creuses sont remplies d'un gaz inerte, à raison de 5 à 30 % p/p des microsphères.

6. Particules revêtues de pigment selon l'une quelconque des revendications précédentes, caractérisées en ce que les microsphères creuses sont remplies d'isobutane.

7. Particules revêtues de pigment selon l'une quelconque des revendications précédentes, caractérisées en ce que les particules de pigment ont une granulométrie de 0,01 à 25 µm, de préférence de 0,02 à 5 µm.

8. Particules revêtues de pigment selon l'une quelconque des revendications précédentes, caractérisées en ce que la granulométrie moyenne des microsphères creuses est au moins double, de préférence au moins décuple, de la granulométrie moyenne des particules de pigment.

9. Particules revêtues de pigment selon l'une quelconque des revendications précédentes, caractérisées en ce qu'elles ont une granulométrie de 5 à 250 µm, de préférence de 5 à 150 µm, tout préférablement de 5 à 80 µm.

10. Particules revêtues de pigment selon l'une quelconque des revendications précédentes, caractérisées en ce que le pigment est présent à raison de 40 à 97 % en poids, de préférence de 75 à 90 % en poids, relativement au poids des particules revêtues de pigment.

11. Particules revêtues de pigment selon l'une quelconque des revendications précédentes, caractérisées en ce que l'agent de couplage est un monoalcoxytitanate ou un produit de coordination phospho-titanate.

12. Particules revêtues de pigment selon l'une quelconque des revendications précédentes, caractérisées en ce que l'agent de couplage est choisi parmi les monoalkyl triisostéaroyl titanates, les monoalkyl diisostéaroyl méthacryloyl titanates, les monoalkyl diméthacryloyl isostéaroyl titanates et les monoalkyl triméthacrylogl titanates dont le groupe alcoxy a de 1 à 20 atomes de carbone.

13. Particules revêtues de pigment selon l'une quelconque des revendications précédentes, caractérisées en ce que l'agent de couplage est l'isopropyl triisostéaroyltitanate.

14. Composition cosmétique caractérisée en ce qu'elle contient des particules revêtues de pigment selon l'une quelconque des revendications 1 à 13.

15. Composition cosmétique selon la revendication 14, caractérisée en ce qu'elle comprend de 0,05 à 90, de préférence de 0,5 à 50 % en poids des particules revêtues de pigment.

16. Composition cosmétique selon la revendication 14 ou 15, caractérisée en ce qu'elle contient aussi des ingrédients cosmétiques classiques, tels que des pigments, des charges, des huiles, des cires, des liants, des humectants, des conservateurs et des parfums.

17. Procédé pour la préparation des particules revêtues de pigment selon l'une quelconque des revendications 1 à 13, caractérisé en ce que les microsphères creuses sont tout d'abord traitées avec 0,01 à 5,0 % en poids d'un agent de couplage silane ou titanate avec agitation continue pour former un mélange homogène auquel on ajoute une suspension fluide du pigment et, après poursuite de l'agitation, on sépare les particules revêtues de pigment.

18. Procédé selon la revendication 17, caractérisé en ce que la suspension fluide du pigment est une suspension aqueuse.

19. Procédé selon la revendication 17 ou 18, caractérisé en ce que l'agent de couplage est pulvérisé sur un lit agité des microsphères creuses.

## Claims

1. Pigment-coated particles each consisting of a polymeric substrate particle, a silane or titanate coupling agent covalently bonded to the external surface of the polymeric substrate particle and a pigment coating covalently bonded to the coupling agent, characterized in that the polymeric substrate particle is a hollow microsphere of a polyyinylidene copolymer.

2. Pigment-coated particles according to Claim 1, characterized in that the density of the hollow microspheres before coating lies between 0.01 and 0.1 g/cm³, preferably between 0.01 and 0.065 g/cm³.

3. Pigment-coated particles according to Claim 1 or 2, characterized in that the size of the hollow microspheres before coating lies between 5 and 40 µm, preferably between 5 and 25 µm.

4. Pigment-coated particles according to any one of the preceding claims, characterized in that the polyyinylidene copolymer is a vinylidene chlorideacrylonitrile copolymer.

5. Pigment-coated particles according to any one of the preceding claims, characterized in that the hollow microspheres are filled with an inert gas, in a proportion of 5 to 30% w/w of the microspheres.

6. Pigment-coated particles according to any one of the preceding claims, characterized in that the hollow microspheres are filled with isobutane.

7. Pigment-coated particles according to any one of the preceding claims, characterized in that the pigment particles have a particle size of 0.01 to 25 µm, preferably of 0.02 to 5 µm.

8. Pigment-coated particles according to any one of the preceding claims, characterized in that the mean particle size of the hollow microspheres is at least double, preferably at least ten times, the mean particle size of the pigment particles.

9. Pigment-coated particles according to any one of the preceding claims, characterized in that they have a particle size of 5 to 250 µm, preferably of 5 to 150 µm, very preferably of 5 to 80 µm.

10. Pigment-coated particles according to any one of the preceding claims, characterized in that the pigment is present in a proportion of 40 to 97% by weight, preferably of 75 to 90% by weight, relative to the weight of the pigment-coated particles.

11. Pigment-coated particles according to any one of the preceding claims, characterized in that the coupling agent is a monoalkoxytitanate or a phosphotitanate coordination product.

12. Pigment-coated particles according to any one of the preceding claims, characterized in that the coupling agent is chosen from monoalkyl triisostearoyltitanates, monoalkyl diisostearoylmethacryloyltitanates, monoalkyl dimethacryloylisostearoyltitanates and monoalkyl trimethacryloyltitanates in which the alkoxy group has from 1 to 20 carbon atoms.

13. Pigment-coated particles according to any one of the preceding claims, characterized in that the coupling agent is isopropyl triisostearoyltitanate.

14. Cosmetic composition, characterized in that it contains pigment-coated particles according to any one of Claims 1 to 13.

15. Cosmetic composition according to Claim 14, characterized in that it comprises from 0.05 to 90, preferably from 0.5 to 50%, by weight of pigment-coated particles.

16. Cosmetic composition according to Claim 14 or 15, characterized in that it also contains conventional cosmetic ingredients, such as pigments, fillers, oils, waxes, binders, humectants, preserving agents and fragrances.

17. Process for the preparation of the pigment-coated particles according to any one of Claims 1 to 13, characterized in that the hollow microspheres are first of all treated with 0.01 to 5.0% by weight of a silane or titanate coupling agent with continuous stirring in order to form a homogeneous mixture to which a fluid suspension of the pigment is added and, after continuation of the stirring, the pigment-coated particles are separated.

18. Process according to Claim 17, characterized in that the fluid suspension of the pigment is an aqueous suspension.

19. Process according to Claim 17 or 18, characterized in that the coupling agent is sprayed onto an agitated bed of the hollow microspheres.

## Patentansprüche

1. Mit Pigment überzogene Teilchen, wobei die Teilchen jeweils aus einer teilchenförmigen Polymergrundlage, einem Silan- oder Titanatkupplungsmittel, das durch kovalente Bindung auf der äußeren Oberfläche der teilchenförmigen Polymergrundlage fixiert ist, sowie einem Pigmentüberzug bestehen, welcher durch kovalente Bindung über das Kupplungsmittel fixiert ist, dadurch **gekennzeichnet**, daß die teilchenförmige Polymergrundlage ein Mikrohohlkügelchen aus einem Polvvinylidencopolymer darstellt.

2. Mit Pigment überzogene Teilchen nach Anspruch 1, dadurch **gekennzeichnet**, daß die Volumenmasse der Mikrohohlkügelchen vor dem Überziehen zwischen 0,01 und 0,1 g/cm³, vorzugsweise zwischen 0,01 und 0,065 g/cm³, beträgt.

3. Mit Pigment überzogene Teilchen nach Anspruch 1 oder 2, dadurch **gekennzeichnet**, daß der Durchmesser der Mikrohohlkügelchen vor dem Überziehen zwischen 5 und 40 µm, vorzugsweise zwischen 5 und 25 µm, beträgt.

4. Mit Pigment überzogene Teilchen nach einem der vorstehenden Ansprüche, dadurch **gekennzeichnet**, daß das Polyvinylidencopolymer ein Copolymer aus Vinyliden/Acrylnitrilchlorid darstellt.

5. Mit Pigment überzogene Teilchen nach einem der vorstehenden Ansprüche, dadurch **gekennzeichnet**, daß die Mikrohohlkügelchen mit einem Inertgas in einem Verhältnis von 5 bis 30 Gew.-% in bezug auf die Mikrokügelchen gefüllt sind.

6. Mit Pigment überzogene Teilchen nach einem der vorstehenden Ansprüche, dadurch **gekennzeichnet**, daß die Mirkohohlkügelchen mit Isobutan gefüllt sind.

7. Mit Pigment überzogene Teilchen nach einem der vorstehenden Ansprüche, dadurch **gekennzeichnet**, daß die Pigmentteilchen eine Korngrößenverteilung (Granulometrie) von 0,01 bis 25 µm, vorzugsweise von 0,02 bis 5 µm, aufweisen.

8. Mit Pigment überzogene Teilchen nach einem der vorstehenden Ansprüche, dadurch **gekennzeichnet**, daß die durchschnittliche Korngrößenverteilung der Mikrohohlkügelchen mindestens den doppelten, vorzugsweise den zehnfachen Wert der durchschnittlichen Korngröße (Granulometrie) der Pigmentteilchen beträgt.

9. Mit Pigment überzogene Teilchen nach einem der vorstehenden Ansprüche, dadurch **gekennzeichnet**, daß sie eine durchschnittliche Korngrößenverteilung von 5 bis 250 µm, vorzugsweise von 5 bis 150 µm, in ganz besonders bevorzugter Weise von 5 bis 80 µm, aufweisen.

10. Mit Pigment überzogene Teilchen nach einem der vorstehenden Ansprüche, dadurch **gekennzeichnet**, daß das Pigment in einem Verhältnis von 40 bis 97 Gew.-%, vorzugsweise von 75 bis 90 Gew.-%, in bezug auf das Gewicht der mit Pigment überzogenen Teilchen vorhanden ist.

11. Mit Pigment überzogene Teilchen nach einem der vorstehenden Ansprüche, dadurch **gekennzeichnet**, daß das Kupplungsmittel ein Monoalkoxytitanat oder ein Koordinationsprodukt von Phosphortitanat darstellt.

12. Mit Pigment überzogene Teilchen nach einem der vorstehenden Ansprüche, dadurch **gekennzeichnet**, daß das Kupplungsmittel unter Monoalkyltriisostearyltitanaten, Monoalkyldiisostearoylmetacryloyltitanaten, Monoalkyldimetacryloylisostearoyltitanaten und Monoalkyltrimetacryloyltitanaten ausgewählt ist, bei denen der Alkoxyrest zwischen 1 und 20 Kohlenstoffatomen aufweist.

13. Mit Pigment überzogene Teilchen nach einem der vorstehenden Ansprüche, dadurch **gekennzeichnet**, daß das Kupplungsmittel Isopropyltriisostearoyltitanat ist.

14. Kosmetische Zusammensetzung, dadurch **gekennzeichnet**, daß sie mit Pigment überzogene Teilchen nach einem der Ansprüche 1 bis 13 enthält.

15. Kosmetische Zubereitung nach Anspruch 14, dadurch **gekennzeichnet**, daß sie 0,05 bis 90, vorzugsweise 0,5 bis 50 Gew.-%, an mit Pigment überzogenen Teilchen aufweist.

16. Kosmetische Zubereitung nach Anspruch 14 oder 15, dadurch **gekennzeichnet**, daß sie zusätzlich noch kosmetische klassische Hilfsstoffe, wie z.B. Pigmente, Trägerstoffe, Öle, Wachse, Bindemittel, Feuchthaltemittel, Konservierungsstoffe und Duftstoffe enthält.

17. Verfahren zur Herstellung von mit Pigment überzogenen Teilchen nach einem der Ansprüche 1 bis 13, dadurch **gekennzeichnet**, daß die Mikrohohlkügelchen zuvor mit 0,01 bis 5 Gew.-% eines Silan- oder Titanatkupplungsmittels unter ständigem Rühren zur Bildung einer homogenen Mischung behandelt wurden, wonach eine flüssige Pigmentsuspension hinzugegeben wird und im Anschluß daran gerührt und die mit Pigment überzogenen Teilchen abgetrennt werden.

18. Verfahren nach Anspruch 17, dadurch **gekennzeichnet**, daß die flüssige Pigmentsuspension eine wäßrige Suspension darstellt.

19. Verfahren nach Anspruch 17 oder 18, dadurch **gekennzeichnet**, daß das Kupplungsmittel in einem bewegten Bett aus Mikrohohlkügelchen pulverisiert wird.
